# EUROPEAN PATENT APPLICATION

(11) **EP 2 025 683 A1**
(43) Date of publication of application: **18.02.2009**
(21) Application number: 07291007.8
(22) Date of filing: 13.08.2007
(51) Int. Cl.: C07K 14/575

(54) **Variants of prolactin as antagonists of its receptor**

(71) Applicant: INSERM (Institut National de la Santé et de la Recherche Medicale), 75013 Paris (FR)
(72) Inventor: The designation of the inventor has not yet been filed
(74) Representative: Vialle-Presles, Marie José

(57) **Abstract**

The invention relates to mammalian prolactin (PRL) variants having a mutation within binding site 2 of PRL, wherein said mutation consists of a substitution of the glycine residue at position 129 (Gly₁₂₉) with any amino acid, with the exception of arginine and proline. These variants are useful as antagonists of mammalian prolactin receptors (PRLR), more particularly of human prolactin receptor (hPRLR).

## Description

The present invention relates to mammalian prolactin (PRL) variants having a G129 substitution within binding site 2 of prolactin, and their use as pure antagonists of mammalian prolactin receptors (PRLRs), more particularly of human prolactin receptor (hPRLR).

Human (h) prolactin receptor (PRLR) antagonists are a new class of potential drugs developed to target prolactin (PRL)-sensitive pathologies that cannot be treated with current inhibitors of the production of hPRL by the pituitary (GOFFIN et al., 2006, Nat. Clin. Pract. Endocrinol. Metab. 2, 571-581). These include dopamine-resistant prolactinomas (i.e. pituitary tumors of PRL-secreting cells), as well as breast cancer, prostate cancer and benign prostate hyperplasia, in which evidence for the tumor growth-promoting actions of autocrine PRL has been emerging within the past decade (GOFFIN et al., 2005, Endocr.Rev. 26, 400-422; CLEVENGER et al., 2003, Endocr.Rev. 24, 1-27). In view of the ubiquitous expression pattern of the PRLR (BOLE-FEYSOT et al., 1998, Endocr.Rev. 19, 225-268), these indications are not necessarily exhaustive and may be extended to other pathologies that remain to be identified or better characterized with respect to the involvement of locally-produced PRL in their etiology.

Due to their mechanism of action involving competition with endogenous hPRL for receptor binding, competitive hPRLR antagonists need to be used in molar excess compared to endogenous hPRL. Therefore, the most promising compounds are anticipated to be those that are devoid of any residual agonistic properties, even at high concentration (BERNICHTEIN et al., 2003, J.Biol.Chem. 278, 35988-35999).

With the aim of developing a PRLR antagonist, the Inventors have first generated an hPRL variant, denoted G129R-hPRL, wherein the helix 3 glycine at position 129 of hPRL has been substituted for an arginine. The biological properties of this variant have been extensively characterized. However, conflicting data have been obtained regarding the antagonistic activity of G129R-hPRL, and the mechanisms underlying this property. G129R-hPRL exhibits no activity in the low-sensitivity HL-5 assay (KINET et al., 1999, J.Biol.Chem. 274, 26033-26043; GOFFIN et al., 1999, Endocrinology 140, 3853-3856), or in signalling studies (immunoblots) (BERNICHTEIN et al., 2001, Endocrinology 142, 3950-3963), while it is able to stimulate the proliferation of Ba/F-LP cells and of rat Nb2 cells (the two most sensitive bioassays for lactogens) to mid- and sub-maximal level, respectively (GOFFIN, et al., 1994, J.Biol.Chem. 269, 32598-32606; GOFFIN et al., 1999, Endocrinology 140, 3853-3856; BERNICHTEIN et al. 2003, Endocrine. 20, 177-190) . Then, it is clear that G129R-hPRL displays residual agonism in some experimental situations, including *in vivo* (GOFFIN et al., 1994, J.Biol.Chem. 269, 32598-32606; GOFFIN et al., 2005, Endocr.Rev. 26, 400-422). Thus, it would be misleading to neglect the residual activity of this mutant otherwise referred to as antagonist. Therefore, it cannot be used therapeutically as a pure antagonist of the prolactin receptor, since it may exert an effect opposite to that expected from an antagonist.

In order to improve the G129R-hPRL properties and abolish its residual agonism, the Inventors have then developed second-generation hPRL antagonists, which are disclosed in PCT Application WO 03/057729. These antagonists consist of hPRL variants having 1) a mutation or a set of mutations within the 14 N-terminal amino acids, said mutation preventing the formation of the Cys4-Cysl1 disulfide bridge and 2) a sterically hindering mutation or set of mutations within binding site 2 wherein said sterically hindering mutations are for instance substitution of at least one residue among Gln₁₂₂, Leu₁₂₅, Ser₂₆, Ala₂₂ or Gly₁₂₉, with a large and/or charged residue such as Tyr, Phe, Asp, Glu, Arg, Lys or Trp.

One of these antagonists, Del1-9-G129R-hPRL (BERNICHTEIN et al., 2003, J.Biol.Chem. 278, 35988-35999), has been thoroughly studied. As highlighted by its name, Del1-9-G129R-hPRL is a hPRL core protein containing two modifications: deletion of the nine N-terminal residues, and substitution of Gly129 with an Arg. In contrast to the previously developed hPRL antagonists (for reviews, GOFFIN et al., 2005, Endocr.Rev. 26, 400-422; GOFFIN et al., 2007, Recent Patents on Endocrine, Metabolic & Immune Drug Discovery 1, 41-52), Del1-9-G129R-hPRL was shown to be devoid of residual agonism in every cell or animal model in which it has been tested to date (BERNICHTEIN et al., 2003, J.Biol.Chem. 278, 35988-35999; MA et al., 2005, Endocrinology 146, 5112-5119; MA et al., 2005, Endocrinology 146, 93-102; EYAL et al., 2007, Biol.Reprod. 76, 777-783; DIOGENES et al., 2006, J.Neurosci. 26, 8126-8136; DAGVADORJ et al., 2007, Endocrinology 148, 3089-3101). Therefore, Del1-9-G129R-hPRL can be considered as a pure PRLR antagonist.

The Inventors have now generated hPRL variants harbouring a single substitution at the Gly129 residue (denoted G129X-hPRL, wherein X is an amino acid substituting Gly129). Seven new substitution variants were generated and compared to the G129R-hPRL and Del1-9-G129R-hPRL variants: G129P-, G129D-, G129N-, G129V-, G129L-, G129Y- and G129F-hPRL variants. These amino acid residues were chosen to explore the influence of different parameters such as volume (P<D<N<V<L<Y<F<R), charge (negative [D], positive [R] or none [P,N,V,L,Y,F]) and polarity (D,N,Y). These variants (i.e., mutants) were analyzed for their (residual) agonistic and antagonistic properties in cell bioassays.

Surprisingly, the Inventors have found that all substitutions (except G129P) led to lower residual agonism than G129R-hPRL. Val, Leu and Phe were in this respect the substitutes leading to the lowest residual agonism.

Accordingly, the present invention provides a method for obtaining a variant of mammalian prolactin, useful as an antagonist of a mammalian prolactin receptor, wherein said method comprises providing a wild-type mammalian prolactin having a glycine residue at position 129 (referring to the numbering of the mature form, devoid of the signal peptide) and substituting said glycine residue by an amino-acid other than glycine, arginine (Arg, R) or proline (Pro, P).

The present invention also provides a variant of mammalian prolactin useful as an antagonist of a mammalian prolactin receptor, obtainable by the method of the invention. Said variant has a mutation consisting of the substitution of the glycine residue at position 129 (Gly₁₂₉) with any amino acid other than glycine, with the exception of arginine (Arg, R) and proline (Pro, P).

According to a preferred embodiment of the present invention, the amino acid substituting Gly₁₂₉ is selected from the group consisting of alanine (Ala, A), asparagine (Asn, N), aspartic acid (Asp, D), cysteine (Cys, C), glutamic acid (Glu, E), glutamine (Gln, Q), histidine (His, H), isoleucine (Ile, I), leucine (Leu, L), lysine (Lys, K), methionine (Met, M), phenylalanine (Phe, F), serine (Ser, S), threonine (Thr, T), tryptophan (Trp, W), tyrosine (Tyr, Y) and valine (Val, V), preferably, Asn, Asp, Leu, Phe, Tyr and Val, more preferably Leu, Phe and Val, and still more preferably Val.

Advantageously, the variants of the invention are variants of human prolactin (hPRL). The sequence of the unprocessed precursor of hPRL is available for instance in the NCBI database under accession number CAA23829, or in the Swiss-Prot database under accession number P01236.

The variants of mammalian prolactin described above only differ from the wild-type mammalian prolactin from which they are derived by the above-defined substitution of the glycine residue at position 129. Optionally, this substitution can be combined with one or more other mutation(s) aiming at further reducing residual agonist activity, for instance mutations preventing the formation of the Cys4-Cysl1 disulfide bridge, such as those disclosed in PCT WO 03/057729. In this regard, preferred variants are those having a mutation or a set of mutations preventing the formation of the Cys4-Cys11 disulfide bridge within the 14 N-terminal amino acids (preferably a deletion of at least the 9 N-terminal and up to the 14 N-terminal residues), and a substitution of the glycine residue at position 129 with a residue selected among Ala, Asn, Cys, Gln, His, Ile, Leu, Met, Ser, Thr, et Val, preferably Asn, Leu, or Val, more preferably Leu or Val, and still more preferably Val.

The present invention also provides polynucleotides encoding the PRL variants of the invention.

Polynucleotides of the invention may be obtained by the well-known methods of recombinant DNA technology and/or of chemical DNA synthesis. These methods also allow to introduce the desired mutations in a naturally occurring DNA sequence.

The invention also provides recombinant DNA constructs comprising a polynucleotide of the invention, such as expression cassettes wherein said polynucleotide is linked to appropriate control sequences allowing the regulation of its transcription and translation in a host cell, and recombinant vectors comprising a polynucleotide or an expression cassette of the invention.

These recombinant DNA constructs can be obtained and introduced in host cells by the well-known techniques of recombinant DNA and genetic engineering.

The invention also comprises a prokaryotic or eukaryotic host cell transformed by a polynucleotide encoding a PRL variant of the invention.

A PRL variant of the invention can be obtained by culturing a host cell containing an expression vector comprising a nucleic acid sequence encoding said PRL variant, under conditions suitable for the expression thereof, and recovering said variant from the host cell culture.

The invention also provides transgenic non-human animals, in particular transgenic non-human mammals, transformed with a polynucleotide encoding a PRL variant of the invention. Suitable methods for the preparation of transgenic animals are for instance disclosed in: Manipulating the Mouse Embryo, 2nd Ed., by HOGAN et al., Cold Spring Harbor Laboratory Press, 1994; Transgenic Animal Technology, edited by C. PINKERT, Academic Press Inc., 1994; Gene Targeting: A Practical Approach, edited by A.L. JOYNER, Oxford University Press, 1995; Strategies in Transgenic Animal Science, edited y G.M. MONASTERSKY and J.M. ROBL, ASM Press, 1995; Mouse Genetics: Concepts and Applications, by Lee M. SILVER, Oxford University Press, 1995.

The invention also relates to a therapeutic composition comprising a PRL variant of the invention, or a polynucleotide encoding said PRL variant, optionally mixed with suitable carriers and/or excipient(s).

For instance, the PRL variants of the invention can further be conjugated to one or more chemical groups, in order to increase their molecular weight. Examples of suitable chemical groups include polyols, such as polyethylene glycol (PEG) or heterologous polypeptides preferably hydrosoluble polypeptides, such as serum albumin or fragments thereof.

Therapeutic compositions of the invention are useful as PRLR antagonists, in particular for treating or preventing diseases involving PRLR-mediated effects, such as tumoral proliferation involving any form of benign or malignant tumor (hyperplasia, dysplasia, neoplasia, adenoma, carcinoma) in any PRL target tissue (breast, prostate, liver, pituitary, lymphocytes, ovary, uterus, pancreas), auto-immune diseases (lupus erythematosus, rheumatoid arthritis), inflammation, hyperprolactinemia, typically, any diseases arising from an overstimulation of the PRLR (hypermastia, reproduction disorders) (BOLE-FEYSOT et al., Endocr. Rev., 1998).

The therapeutic compositions of the invention can be administered in various ways:
They can be used systemically or locally. A preferred route of administration is the parenteral route, including for instance intramuscular, subcutaneous, intravenous, intraperitoneal, or local intratumoral injections.
The oral route can also be used, provided that the composition is in a form suitable for oral administration, able to protect the active principle from the gastric and intestinal enzymes.
In the case wherein the therapeutic composition includes a polynucleotide encoding a PRL variant of the Invention, said nucleotide is generally inserted in an expression cassette allowing its expression in a target organ or tissue.
The expression cassette can be directly transferred in the cells as naked DNA, or placed in an appropriate vector, such as a viral vector, for instance an adenovirus derived vector.
Gene transfer can be performed *ex vivo* on cells removed from the subject to be treated and thereafter re-implanted into said subject, or can be performed by direct administration of the nucleic acid to said subject.
The choice of the method of transfer and/or of the vector depends on the target organ or tissue, and/or on whether a short-time expression (transient expression) or a more stable expression is wanted.
Since the PRL variants of the Invention have a lower affinity for the PRL receptor than native PRL, the amount administered will be chosen in order to supply a large excess of PRL variant over endogenous PRL in the blood and/or target tissue. On the other hand, due to the lack of residual agonist activity of PRL variants of the invention, high doses thereof can be administered, without risk of unwanted agonist effects. In most of cases, an amount of PRL variant resulting in a 10 to 100-fold excess over endogenous PRL will be suitable. If necessary, an amount of PRL variant resulting in a 1000-fold excess or more over endogenous PRL can be administered.
The present invention will be further illustrated by the following additional description, which refers to examples illustrating the properties of hPRL antagonists of the invention, as well as to the appended figure.

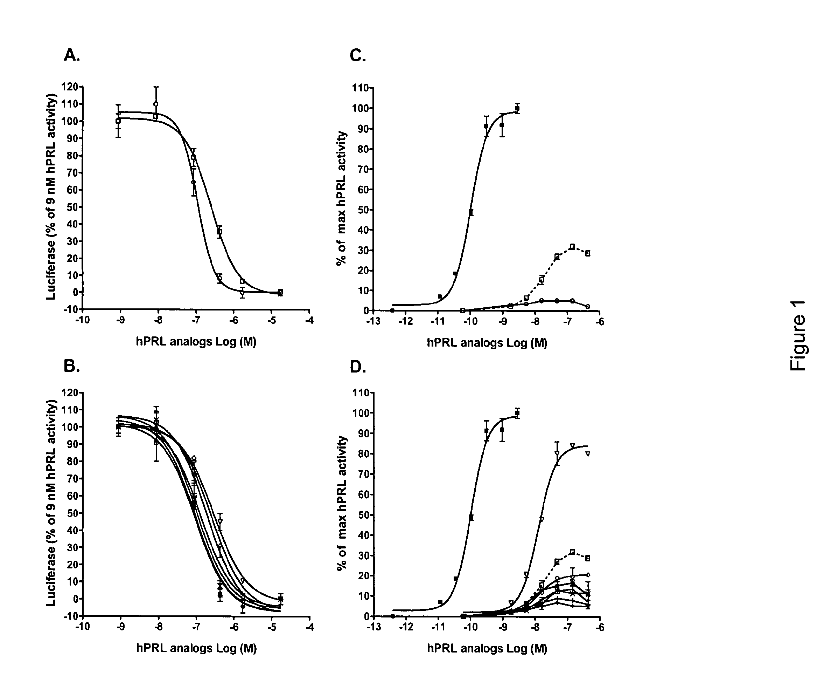

### Figure 1. Antagonism and residual agonism displayed by hPRL variants.

Representative experiments (performed in triplicate) of antagonism assays against 9 nM hPRL using HL-5 cells (A and B), and agonism assays using Ba/F-LP cells (C and D) are shown for G129R-containing mutants (A and C) and the other G129 variants (B and D). Data are expressed as percentage of the maximal activity of hPRL in each panel. Values of IC₅₀ (antagonism) and maximal agonistic potency of all variants are reported in Table 2. *Symbols*: ■, hPRL; ○, Del1-9-G129R-hPRL; □ (dotted line), G129R-hPRL; ∇, G129P-hPRL; ◇, G129D-hPRL; ×, G129F-hPRL; ◆, G129V-hPRL; +, G129L-hPRL; ∗, G129N-hPRL; G129Y-hPRL.

It should be understood however that these examples are given only by way of illustration of the invention and do not constitute in any way a limitation thereof.

### EXAMPLE: BIOACTIVITY OF GLY₁₂₉X-hPRL VARIANTS

With respect to the Gly129 mutations, seven substitution variants were generated and compared to the G129R-hPRL and Del1-9-G129R-hPRL variants (GOFFIN et al., 1994, J.Biol.Chem. 269, 32598-32606): P, D, N, V, L, Y and F. These residues were chosen to explore the influence of different parameters such as volume (P<D<N<V<L<Y<F<R), charge (negative [D], positive [R] or none [P,N,V,L,Y,F],) and polarity (D,N,Y).

### 1) Experimental Procedures

### Materials

Culture media, fetal calf serum (FCS), geneticin (G-418), trypsin and glutamine were purchased from Gibco-Invitrogen (Grand Island, NY, USA). Luciferin and cell lysis buffer were purchased from Promega (Madison, WI), and luciferase activity was measured in relative light units (RLU) using a Lumat LB 9501 (Berthold, Nashua, NH). Iodogen was purchased from Sigma-Aldrich (Saint Quentin Fallavier, France), and carrier-free Na[¹²⁵I] was obtained from GE-Healthcare (Buckinghamshire, UK). Oligonucleotides were purchased from Eurogentec (Liège, Belgium). Chemicals were purchased from Sigma-Aldrich (Saint Quentin Fallavier, France), VWR (Fontenay-sous-Bois, France) or Merck (Darmstadt, Germany).

### Site directed mutagenesis

Expression plasmids encoding wild-type (WT) hPRL, G129R-hPRL and Del1-9-G129R-hPRL were produced as described in BERNICHTEIN et al., 2003 (J.Biol.Chem. 278, 35988-35999) and GOFFIN et al., 1994 (J.Biol.Chem. 269, 32598-32606). The hPRL expression plasmid was used as template for generating the expression plasmids for G129X mutants (variants), using the QuikChange II Mutagenesis kit from Stratagene (La Jolla, CA). Sequences of forward and reverse (complementary) primers are given in Table 1 below, with mutated codons underlined.

**Table 1: Mutagenesis primers**

| PRL variants | Primers |
|---|---|
| G129P | Forward (SEQ ID NO: 1): 5'-CAAACGGCTTCTAGAGGCCATGGAGCTGATAGTC-3' Reverse (SEQ ID NO: 2): 5'-GACTATCAGCTCCATGGCCTCTAGAAGCCGTTTG-3' |
| G129D | Forward (SEQ ID NO: 3): 5'-CAAACGGCTTCTAGAGGACATGGAGCTGATAGTC-3' Reverse (SEQ ID NO:4): 5'-GACTATCAGCTCCATGTCCTCTAGAAGCCGTTTG-3' |
| G129F | Forward (SEQ ID NO: 5): 5'-CAAACGGCTTCTAGAGTTCATGGAGCTGATAGTC-3' Reverse (SEQ ID NO: 6): 5'-GACTATCAGCTCCATGAACTCTAGAAGCCGTTTG-3' |
| G129L | Forward (SEQ ID NO: 7): 5'-CAAACGGCTTCTAGAGCTGATGGAGCTGATAGTC-3' Reverse (SEQ ID NO: 8): 5'-GACTATCAGCTCCATCAGCTCTAGAAGCCGTTTG-3' |
| G129N | Forward (SEQ ID NO: 9): 5'-CAAACGGCTTCTAGAGAACATGGAGCTGATAGTC-3' Reverse (SEQ ID NO: 10): 5'-GACTATCAGCTCCATGTTCTCTAGAAGCCGTTTG-3' |
| G129Y | Forward (SEQ ID NO: 11): 5'-CAAACGGCTTCTAGAGTACATGGAGCTGATAGTC-3' Reverse (SEQ ID NO: 12): 5'-GACTATCAGCTCCATGTACTCTAGAAGCCGTTTG-3' |
| G129V | Forward (SEQ ID NO: 13): 5'-CAAACGGCTTCTAGAGGTCATGGAGCTGATAGTC-3' Reverse (SEQ ID NO: 14): 5'-GACTATCAGCTCCATGACCTCTAGAAGCCGTTTG-3' |

For all steps, recommendations of the manufacturer were strictly followed. After transformation, *E. coli* BL21(DE3) colonies were analysed for their DNA content; plasmids were sequenced to verify the presence of the expected mutations.

### Production and purification of hPRL variants.

Recombinant WT hPRL and hPRL variants were overexpressed in 0.5-1L cultures of E. *coli* BL21(DE3) and purified as described previously in PARIS et al. (1990, Biotechnol.Appl.Biochem. 12, 436-4491990), with minor modifications. Briefly, when the OD₆₀₀ of bacterial cultures reached 0.7-0.9, overexpression was induced using 2 mM IPTG for 4h (OD₆₀₀=2-2.5 after 4 h). Cells were broken using high pressure (French Press). Proteins were overexpressed as insoluble inclusion bodies, which were solubilized in 8 M urea (5 min at 55°C, then 2h at room temperature) and refolded by continuous dialysis (72h, 4°C) against 100 volumes of 25 mM NH₄HCO₃, pH 8.6. Solubilized proteins were centrifuged, then loaded onto a HiTrap Q anion exchange column (GE-Healthcare) equilibrated in 25 mM NH₄HCO₃, pH 8.6. Prolactin (WT and variants) was eluted along a NaCl gradient (0-500 mM), and the major peak was collected, quantified and kept frozen until use. Purity of the various hPRL variant batches was >95% as judged from SDS-PAGE analysis.

### Binding assays.

Binding affinities of hPRL variants were determined using cell homogenates of HEK 293 cells stably expressing the human PRLR (so-called HL-5 clone), following a procedure previously described in KINET et al., 1999 (J.Biol.Chem. 274, 26033-26043).

Human PRL was iodinated using Iodogen, and binding assays were performed overnight at room temperature using 150-300 µg cell homogenate protein in the presence of 20,000 to 30,000 cpm [¹²⁵I]-hPRL and increasing concentrations of unlabeled competitor (WT or mutated hPRL). Results presented are representative of at least three independent experiments performed in duplicate. The relative binding affinities of G129R-, G129P-, G129D-, G129N-, G129V-, G129L-, G129Y- and G129F-hPRL variants were calculated with respect to that of WT hPRL based on their IC₅₀.

### Cell-based bioassays.

The biological properties of hPRL variants were analyzed using two homologous bioassays that were developed for human lactogens (BERNICHTEIN et al., 2003, Endocrine. 20, 177-190). The transcriptional assay involved HEK 293 cells stably expressing the hPRLR and the LHRE (lactogenic hormone response element)-luciferase reporter gene (HL-5 clone); it was used to determine the antagonistic properties by competing a fixed concentration (9 nM) of WT hPRL with increasing amounts of each variant (as described in KINET et al. 1999, J.Biol.Chem. 274, 26033-26043; BERNICHTEIN et al., 2003, Endocrine. 20, 177-190; BERNICHTEIN et al., 2001, Endocrinology 142, 3950-3963). A proliferation assay was also performed to determine the residual agonistic activity of the antagonists, using Ba/F-03 cells stably expressing the human PRLR (referred to as Ba/F-LP cells); this assay has been shown to be much more sensitive than the HL-5 clone to reveal low-level agonistic effects (BERNICHTEIN et al., 2003, J.Biol.Chem. 278, 35988-35999; BERNICHTEIN et al., 2003, Endocrine. 20, 177-190; GLEZER et al., 2006, J.Clin.Endocrinol.Metab 91, 1048-1055). These assays were run as follows.

### i) Antagonism bioassay (HL-5 cells)

The HL-5 clone was cultured in DMEM medium supplemented with 10% FCS, 2 mM glutamine, 50 U/mL penicillin, 50 µg/mL streptomycin, and 700 µg/mL G-418 (for clonal selection). The assay was performed in 96-well plates using 50,000 cells/100 µL/well in medium containing only 0.5% FCS. Cells were allowed to adhere for 6h, then 100 µL [2x] hormones diluted in 0.5% FCS medium were added to each well. After overnight stimulation, cells were lysed (50 µL lysis buffer, Promega), then luciferase activity contained in 10-20 µL cell lysate was counted for 10 seconds. To avoid inter-assay variations, all variants to be compared were systematically tested in the same experiment, and data obtained in one experiment representative of at least three experiments performed in duplicate are shown. In antagonism experiments, a mix of 50 µL of [4x] hormone variants combined with 50 µL of [4x] WT hPRL (to obtain a final concentration of 9 nM) were added. Experimental data were plotted using the GraphPad software.

### ii) Agonism bioassay (Ba/F3-LP cells).

Ba/F3-LP cells were maintained in RPMI 1640 medium supplemented with 10% heat-inactivated FCS, 2 mM glutamine, 50 U/mL penicillin, 50 µg/mL streptomycin, 500-1,000 µg/mL G-418, and 10 ng/mL WT hPRL instead of Interleukin-3 (non transfected Ba/F03 cells are dependent on that cytokine for survival/growth). For the assay, cells were starved for 6 h in 1-2% FCS RPMI medium (with additives), then distributed in 96-well plates at a density of 50,000 cells/100 µL/well in the same medium (excluding hormones). One hundred µL of [2x] hormones were added to each well. Cell proliferation was monitored after 2 days of hormonal stimulation using 10-15 µl of cell proliferation reagent WST-1 (Roche). Experiments were performed at least three times in triplicate. Experimental data were plotted using the GraphPad software.

### 2) Results

### Binding studies on the full-length transmembrane PRLR

The affinity of G129R-hPRL is ∼1 order of magnitude lower than WT hPRL (IC₅₀=19 ± 5 nM), confirming previous data obtained by KINET *et al.,* 1999. None of the G129X substitutions significantly modified the affinity for the human receptor compared to G 129R-hPRL (data not shown).

### Cell-based bioassays

### i) Antagonism.

The antagonistic properties of all hPRL variants were investigated using HL-5 luciferase assay. All data presented on Figures 1A and B were obtained by competing the same concentration of hPRL (9 nM). All mutants harbouring a mutation of Gly129 exhibited antagonism in this assay. With respect to G129R-containing mutants, Del1-9-G129R-hPRL was slightly, but repeatedly, a more potent antagonist than G129R-hPRL (IC₅₀: 120 ± 43 nM versus 200 ± 26 nM), confirming previous observations (BERNICHTEIN et al., 2003, J.Biol.Chem. 278, 35988-35999). Other substitutions at position Gly129 gave rise to parallel curves of antagonism (Figure. 1B), with similar IC₅₀ that varied by less than 3-fold between the most (G129V or G129F) and the less (G129P or G129D) potent antagonists. All data are summarized in Table 2 below.

**Table 2: Agonistic and antagonistic properties of hPRL variants**

| G129 variants | Antagonism HL-5 cells IC₅₀ (nM) | Agonism Ba/F-LP cells (% of hPRL max activity) |
|---|---|---|
| G129P-hPRL | 340 ± 9 | 90.4 ± 7.4 |
| G129R-hPRL | 200 ± 26 | 45.5 ± 12.8 |
| G129N-hPRL | 130 ± 19 | 26.4 ± 11.8 |
| G129Y-hPRL | 190 ± 70 | 24.3 ± 10.8 |
| G129D-hPRL | 340 ± 50 | 23.6 ± 4.1 |
| G129F-hPRL | 110 ± 41 | 19.1 ± 6.2 |
| G129L-hPRL | 160 ± 34 | 16.8 ± 7.2 |
| G129V-hPRL | 110 ± 12 | 14.2 ± 5.0 |
| Del1-9-G129R-hPRL | 120 ± 43 | 8.3 ± 3.6 |

### ii) Residual agonism.

G129R-hPRL displayed <2% of hPRL maximal activity in the HL-5 assay which used to be considered as negligible (background). Similarly, all G129 mutants failed to induce detectable luciferase response, with the exception of G129P-hPRL for which a low activity was reproducibly observed (data not shown).
The Ba/F-LP proliferation assay is much more sensitive than the HL-5 luciferase assay, therefore allowing measurements of residual agonism of variants otherwise referred to as PRLR antagonists (BERNICHTEIN et al., 2003, Endocrine. 20, 177-190). In sharp contrast to the HL-5 assay, G129R-hPRL exhibited 30-50% of maximal hPRL activity on cell proliferation (Figure 1C) (BERNICHTEIN et al., 2003, Endocrine. 20, 177-190), although the dose-response curve was displaced to the right by two orders of magnitude compared to full agonists (ED₅₀ ∼10 nM, and maximal activity achieved at ∼100 nM). All single G129 mutants exhibited dose-response curves with ED₅₀ similar to G129R-containing mutants (Figure 1D and Table 2 above). While G129P-hPRL achieved sub-maximal activity in this assay (∼90% of WT hPRL), all other variants were less active than G129R-hPRL. Variant G129V-hPRL displayed virtually no activity, as Del1-9-G129R-hPRL.

### 3) Conclusion

All Gly129 mutants exhibited similar antagonism in the HL-5 assay (IC₅₀ varying by less than 3-fold, Figure 1B), confirming that any residue other than Gly at position 129 transforms hPRL into an antagonist. However, all substitutions (except G129P) led to lower residual agonism than G129R (Figure 1D). Surprisingly, Arg is actually one of the less efficient substitutions of Gly129 when attempting to generate a pure antagonist. Val, Leu and Phe were in this respect the substitutes leading to the lowest residual agonism.

## Claims

1. An antagonist of a mammalian prolactin receptor **characterised in that** it consists of a variant of mammalian prolactin having a mutation consisting of the substitution of the glycine residue at position 129 (Gly₁₂₉) with any amino acid other than glycine, with the exception of arginine (Arg, R) and proline (Pro, P).

2. An antagonist according to claim 1, **characterised in that** said amino acid is selected from the group consisting of alanine, asparagine, aspartic acid, cysteine, glutamic acid, glutamine, histidine, isoleucine, lysine, methionine, tyrosine, serine, threonine, tryptophan.

3. An antagonist according to claim 1, **characterised in that** the amino acid is selected from the group consisting of leucine, phenylalanine and valine.

4. An antagonist according to any of claims 1 to 3, **characterised in that** it is a variant of human prolactin.

5. A polynucleotide encoding a variant of prolactin of any one of claims 1 to 4.

6. An expression cassette comprising a polynucleotide of claim 5.

7. A recombinant vector comprising a polynucleotide of claim 5.

8. A host cell transformed by a polynucleotide of claim 5.

9. A transgenic non-human mammal transformed with a polynucleotide of claim 5.

10. A therapeutic composition comprising an antagonist of a mammalian prolactin receptor according to any of claims 1 to 4 or a polynucleotide of claim 5.

11. Use of an antagonist of a mammalian prolactin receptor according to any of claims 1 to 4, or of a polynucleotide of claim 5 for obtaining a therapeutic composition for treating or preventing a disease involving PRL- mediated effects.


SEQUENCE LISTING

<110>  INSTITUT NATIONAL DE LA SANTE ET DE LA RECHERCHE MEDICALE
       (INSERM)

<120>  ANTAGONISTS OF MAMMALIAN PROLACTIN RECEPTOR

<130>  MJPmad598/100

<160>  14

<170>  PatentIn version 3.3

<210>  1
<211>  34
<212>  DNA
<213>  Artificial

<220>
<223>  Primer

<400>  1
caaacggctt ctagaggcca tggagctgat agtc                                34


<210>  2
<211>  34
<212>  DNA
<213>  Artificial

<220>
<223>  Primer

<400>  2
gactatcagc tccatggcct ctagaagccg tttg                                34


<210>  3
<211>  34
<212>  DNA
<213>  Artificial

<220>
<223>  Primer

<400>  3
caaacggctt ctagaggaca tggagctgat agtc                                34


<210>  4
<211>  34
<212>  DNA
<213>  Artificial

<220>
<223>  Primer

<400>  4
gactatcagc tccatgtcct ctagaagccg tttg                                34

<210>  5
<211>  34
<212>  DNA
<213>  Artificial

<220>
<223>  Primer

<400>  5
caaacggctt ctagagttca tggagctgat agtc                                    34


<210>  6
<211>  34
<212>  DNA
<213>  Artificial

<220>
<223>  Primer

<400>  6
gactatcagc tccatgaact ctagaagccg tttg                                    34


<210>  7
<211>  34
<212>  DNA
<213>  Artificial

<220>
<223>  Primer

<400>  7
caaacggctt ctagagctga tggagctgat agtc                                    34


<210>  8
<211>  34
<212>  DNA
<213>  Artificial

<220>
<223>  Primer

<400>  8
gactatcagc tccatcagct ctagaagccg tttg                                    34


<210>  9
<211>  34
<212>  DNA
<213>  Artificial

<220>
<223>  Primer

<400>  9
caaacggctt ctagagaaca tggagctgat agtc                                    34


<210>  10

<211>  34
<212>  DNA
<213>  Artificial

<220>
<223>  Primer

<400>  10
gactatcagc tccatgttct ctagaagccg tttg                                    34


<210>  11
<211>  34
<212>  DNA
<213>  Artificial

<220>
<223>  Primer

<400>  11
caaacggctt ctagagtaca tggagctgat agtc                                    34


<210>  12
<211>  34
<212>  DNA
<213>  Artificial

<220>
<223>  Primer

<400>  12
gactatcagc tccatgtact ctagaagccg tttg                                    34


<210>  13
<211>  34
<212>  DNA
<213>  Artificial

<220>
<223>  Primer

<400>  13
caaacggctt ctagaggtca tggagctgat agtc                                    34


<210>  14
<211>  34
<212>  DNA
<213>  Artificial

<220>
<223>  Primer

<400>  14
gactatcagc tccatgacct ctagaagccg tttg                                    34